# EUROPÄISCHE PATENTSCHRIFT

(11) **EP 1 608 259 B1**
(45) Veröffentlichungstag und Bekanntmachung des Hinweises auf die Patenterteilung: **01.02.2017**
(21) Anmeldenummer: 04722809.3
(22) Anmeldetag: 24.03.2004
(51) Int. Cl.: A61B 3/00, G02B 21/06

(54) **BELEUCHTUNGS- UND BESTRAHLUNGSEINHEIT FÜR OPHTHALMOLOGISCHE GERÄTE**
ILLUMINATION AND IRRADIATION UNIT FOR OPHTHALMOLOGIC DEVICES
UNITE D'ECLAIRAGE ET D'IRRADIATION POUR DES APPAREILS OPHTALMOLOGIQUES

(30) Priorität: 02.04.2003 DE 10314944
(43) Veröffentlichungstag der Anmeldung: 28.12.2005
(73) Patentinhaber: Carl Zeiss Meditec AG, 07745 Jena (DE)
(72) Erfinder: LUTHER, Egon, 07751 Cospeda (DE); KOSCHMIEDER, Ingo, 07743 Jena (DE); BUCHHEISTER, Jan, 07751 Jena (DE); MÖHR, Falk, 07751 Jena (DE)
(74) Vertreter: Beck, Bernard
(86) Internationale Anmeldenummer: PCT/EP2004/003089
(87) Internationale Veröffentlichungsnummer: WO 2004/086960

(56) Entgegenhaltungen:
- WO-A-02/26121
- DE-A1- 19 812 050
- US-A- 5 963 300
- US-A1- 2002 133 145

## Beschreibung

Die vorliegende Erfindung betrifft eine Anordnung zur Erzeugung einer variablen Beleuchtung für die Diagnose und Therapie, insbesondere am menschlichen Auge. Das beleuchtete Objekt kann dabei sowohl ein künstliches Objekt als auch ein biologisches Gewebe, beispielsweise ein Auge sein. Bei einem Auge ist die Bestrahlung der Augenlinse aber auch anderer Augenabschnitte wie Kornea oder Retina möglich.

Insbesondere kann die Lösung auch für die Feinabstimmung von in ein Auge eingebrachten optisch wirksame Formteile, z. B. Linsen aus Kunststoff eingesetzt werden, wenn diese gemäß WO 00/41650 und/oder WO 01/71411 aus verschiedenen photosensitiven Kunststoffen bestehen. Bei dieser Art von Linsen werden durch Bestrahlung Polymerisationsvorgänge angeregt, die irreversible chemische Veränderungen der Linsen-Substanz zur Folge haben. Durch diese Vorgänge können der Brechungsindex und/oder das Transmissionsverhalten für die sichtbare Nutzstrahiung bzw. die geometrische Form der Linsen definiert verändert und dadurch ein fehlerreduziertes Sehen ermöglicht werden.

In den Patentschriften WO 00/41650 und WO 01/71411 werden Linsen, insbesondere Intraokularlinsen (IOL) beschrieben, bei denen durch Bestrahlung die Polymerisation einer in der Linse enthaltenen Polymermatrix angeregt und dadurch der Brechungsindex oder die Form der Gesamtlinse verändert werden kann. Bei implantierten IOL besteht das Problem, dass bei ca. der Hälfte der Patienten eine akzeptable Sehleistung nur durch ein zusätzliches Korrektionsmittel wie eine Brille oder Kontaktlinsen erreicht werden kann. Dies resultiert aus Messfehlern bei der Augenvermessung, Abweichungen bei der Positionierung der IOL und/oder durch den Wundheilungsprozess. Mit den beschriebenen IOL wird durch eine gezielte Bestrahlung eine Korrektur der bereits implantierten IOL ermöglicht, indem durch Änderung des Brechungsindex, der Transmissionseigenschaften oder Änderungen der Form eine Anpassung an die tatsächlichen Gegebenheiten erfolgt. Die Bestrahlung der IOL zur Anregung des Polymerisationsvorganges erfolgt vorzugsweise mittels Laserquellen oder Lampen, die einen hohen UV-Anteil des Lichtes aussenden. Hierbei dient als Bestrahlungsquelle ein He/Cd-Laser bzw. eine Xe/Hg-Lampe. Die eventuell erforderlichen Beleuchtungsstrukturen werden in der Regel mit Hilfe mechanischer Blenden und/oder Filtern erzeugt.

Die in WO 02/26121 beschriebene Lösung betrifft ein Verfahren und eine Anordnung zur Bestrahlung lichtjustierbarer Linsen, vorzugsweise der in das Auge implantierten Kunststofflinsen. Die für die Bestrahlung erforderlichen Muster und Profile, sowie die Zeitdauer werden anhand zuvor gemessener Daten ermittelt und über einen Strahlteiler in den Beleuchtungsstrahlengang eingeblendet. Durch eine Wellenfront-Analyse kann die durch die Bestrahlung erreichte Wirkung kontrolliert werden.

Die WO 2002/26 121 A1 beschreibt ein Verfahren und ein Gerät, mit dem eine einstellbare Linse mit Licht bestrahlt wird, die zum Beispiel in ein menschliches Auge impiantieri wurden ist. Dazu wird die momentane Aberration gemessen und die Werte für eine entsprechende Korrektur bestimmt. Im Anschluss erfolgt die modifizierte Bestrahlung der implantierten Linse mit einem definierten Intensitätsmuster, welches in Bezug auf die Intensität und Dauer gesteuert und überwacht wird. Zur Realisierung der definierten Intensitätsmuster können hierbei Apodisierungsfilter, ultraviolette oberflächenstrahlende Laser mit Vertikalhohlraum (VCSEL) oder dynamische Lichtmodulatoren (z. B. LCD oder DMD) Verwendung finden.

Ein Verfahren und eine Vorrichtung zur Untersuchung des Augenhintergrundes wird in DE 100 42 718 beschrieben. Um bei einer schonenden Beleuchtung eine möglichst kontinuierliche Bildaufzeichnung zu gewährleisten, wird über Filteranordnungen wechselweise ein infraroter bzw. ein sichtbarer Lichtanteil des Beleuchtungslichtes durchgelassen. Nachteilig wirken sich dabei die nicht zusammenfallenden optischen Achsen von Beobachtungsstrahlengang und Beleuchtungsstrahlengang aus.

Die DE 199 43 735 A1 beschreibt ein Verfahren und eine Vorrichtung zur gezielten Bestrahlung eines Auges mittels Licht aus dem sichtbaren und/oder nahinfraroten Wellenlängenbereich. Durch die Bestrahlung werden irreversible chemische Veränderungen der Augenlinsen-Substanz hervorgerufen, die eine Veränderung des Brechungsindex und/oder der Transmissionseigenschaften für die sichtbare Nutzstrahlung zur Folge haben und dadurch ein fehlerreduziertes Sehen ermöglichen. Die erfolgreiche Behandlung setzt dabei eine möglichst engmaschige und vollflächige Bestimmung der Verteilung der Brechkraft des zu behandelnden Auges voraus. Aus diesen Werten werden die nach der Behandlung gewünschte Brechkraftverteilung und die dafür erforderlichen Daten der Bestrahlung ermittelt. Als nachteilig wirkt sich bei dieser Lösung aus, dass die Bestrahlung in der Regel nur punktweise nacheinander erfolgen kann und das Behandlungsverfahren dadurch zeitintensiv ist. Für die Dauer der Behandlung ist deshalb eine Fixierung des Augapfels unerlässlich.

In der DE 198 12 050 A1 sind ein Verfahren und eine Anordnung zur Beleuchtung bei einem Augenmikroskop beschrieben. Die verschiedensten Leuchtmarkengeometrien werden mit Hilfe opto-elektronischer Bauelemente erzeugt und auf den Augenvorder- oder Hintergrund projiziert. Diese Lösung dient der allgemeinen Untersuchung des Auges. Eine Anordnung zur Erzeugung von Schnittbildern in transparenten Medien ist in der noch unveröffentlichten Schrift DE 101 55 464.8 vorgesehen. Ebenfalls noch nicht veröffentlicht ist ein ophthalmologisches Untersuchungsgerät mit dem neben einer allgemeinen Augenuntersuchung auch eine perimetrische Untersuchung ermöglicht wird (DE 101 51 314.3). Die Lösungen dieser beiden Schriften sehen ebenfalls die Verwendung opto-elektronischer Bauelemente zur Erzeugung der Beleuchtungsmarken und - muster vor.

In der US 2002/0 133 145 A1 wird eine Laserspaltlampe beschrieben, die mit einem Applikator verbunden ist und sowohl über Mitteln zur Lenkung der Strahlung eines Wirkstrahls auf den zu behandelnden Ort, als auch über Mitteln zur Erzeugung eines Zielstrahls zur Anzielung des zu behandelnden Ortes im oder am Auge verfügt. Die offenbarte Laserspaltlampe verfügt weiterhin über ein Spaltlampenmikroskop sowie Steuer-, Regel- und Überwachungseinrichtungen, wobei Veränderung der Intensität und des Durchmessers des zur Behandlung benutzten Wirkstrahlflecks im Applikator vorgenommen werden. Die beschriebene Lösung dient der Diagnose und Therapie auf dem Gebiet der Ophthalmologie. Mit der beschriebenen Lösung wird eine kompakte Realisierung eines therapeutischen Laserinstruments erreicht, wobei insbesondere auch eine Reduzierung optischer Koppelverluste durch die Anordnung interner Strahlungsquellen eintritt.

Eine Vorrichtung und ein Verfahren zur objektiven Messung und Korrektur optischer Systeme, wie beispielsweise des menschlichen Auges, unter Verwendung einer Wellenfrontanalyse werden in der WO 2001/85 016 A1 beschrieben. Die Messungen der Wellenfronten werden vorgenommen, um eine Korrektur mit dem Laser-Ablationssystem vornehmen zu können. Dazu verfügt die Lösung über einen entsprechenden Laser zum Abtragen der Hornhaut. In Abhängig von der gewünschten Therapie, wie beispielsweise eine Ablation von Hornhautgewebe oder dem Implantieren einer synthetischen Linse kann die Menge des bei jeder (x, y) Koordinate zu entfernenden Materials direkt aus den Messwerten der Wellenfrontanalyse berechnet werden, wenn der Brechungsindex des fraglichen Materials bekannt ist. Bei vielen Verfahren kann eine Wellenfrontanalyse auch während einer Prozedur zur Bereitstellung von Rückkopplungsinformation bezüglich des richtigen Endpunktes der Prozedur wiederholt durchgeführt werden.

Derartige Anordnungen haben jedoch die Nachteile, dass bei einer seitlichen Beleuchtung mitunter nicht das gesamte Beobachtungsgebiet ausgeleuchtet wird, oder dass es durch die Verwendung von Linksystemen zur Einkopplung der Beleuchtungsstrahlung zu Abbildungsfehlern kommen kann. Außerdem sind für die Einkopplung der Beleuchtungsstrahlung mitunter aufwendige technische Lösungen erforderlich.

Der vorliegenden Erfindung liegt die Aufgabe zugrunde eine Einheit für ophthalmologische Geräte zum Beleuchten/Bestrahlen des menschlichen Auges zum Zwecke der Beobachtung und/oder Behandlung zu entwickeln. Dabei soll an dem bewährten Design der ophthalmologischen Geräte festgehalten und deren Aufbau nicht wesentlich komplizierter gestaltet werden.

Erfindungsgemäß wird die Aufgabe durch die Merkmale der unabhängigen Ansprüche gelöst. Bevorzugte Weiterbildungen und Ausgestaltungen sind Gegenstand der abhängigen Ansprüche.

Die vorliegende Erfindung betrifft eine Beleuchtungs- und Bestrahlungseinheit zur Erzeugung verschiedener Marken, Muster und Profile und ist somit sowohl für die Diagnose als auch die Therapie in der Ophthalmologie einsetzbar. Die Beleuchtungseinheit ist dabei für verschiedene ophthalmologische Geräte geeignet.

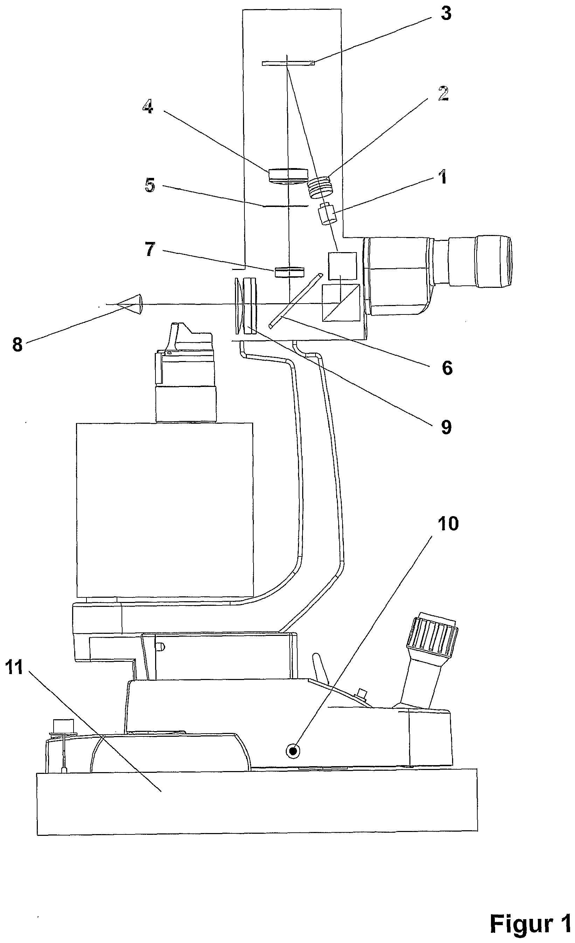

Die technische Lösung wird nachfolgend anhand eines Ausführungsbeispiels beschrieben. Dazu zeigt
- Figur 1:: den Prinzipaufbau der vorgeschlagenen Beleuchtungs- und Bestrahlungseinheit bei einer Spaltlampe.

Die Beleuchtungseinheit für ophthalmologische Geräte besteht aus einer Beleuchtungsquelle 1, Mitteln zur Erzeugung, Überwachung und Kontrolle von Beleuchtungsmustern und/oder Profilen, Mitteln zur Einkopplung des Beleuchtungslichtes in den parallelen Strahlengang des Beobachtungssystems des ophthalmologischen Gerätes sowie einer zentralen Steuer- und Auswerteeinheit.

In **Figur 1** ist eine Beleuchtungs- und Bestrahlungseinheit für eine Spaltlampe dargestellt, bei der die Beleuchtungsquelle **1** ein schmalbandiges Licht im kurzwelligen Bereich um 365nm erzeugt. Das von der Beleuchtungsquelle **1,** beispielsweise einer Bogenlampe erzeugte Lichtbündel wird mit der Kondensorgruppe **2** auf die Mittel zur Erzeugung von Beleuchtungsmustern und/oder Profile gelenkt. Diese Mittel können hierbei feste oder austauschbare, optische Filter und/oder Blenden, oder aber auch opto-elektronische Lichtmodulatoren **3** sein. Als opto-elektronische Lichtmodulatoren **3** können hierbei beispielsweise ein Mikrodisplay vom DMD-Typ (digital micromirror device) bzw. ein reflektierendes Mikrodisplay vom LCOS-Typ (liquid crystal on silicon) zum Einsatz kommen. Es sind aber auch opto-elektronischen Lichtmodulatoren **3** vom transmissiven LCD-Typ (liquid crystal display), selbstleuchtenden LED-Typ (light emitting diode) oder OLED-Typ (organic light emitting diode) einsetzbar. Die Steuerung der opto-elektronische Lichtmodulatoren **3,** die auf transmissiver oder reflektiver Basis arbeiten können, erfolgt durch eine Steuereinheit (nicht dargestellt). Mit Hilfe der genannten Mittel lassen sich beliebige Muster, Profile und Verteilungen zum Erzeugen der unterschiedlichsten Wirkungen erzeugen. Die Beleuchtungsstrahlung kann durch optische Filter **4** und/oder Blenden **5** in seinem spektralen und räumlichen Bereich beeinflusst werden. Die spektrale Bandbreite der Beleuchtungsstrahlung wird beispielsweise durch geeignete Filter **4** auf 365nm +/- 5nm begrenzt.

Als Mittel zur Einkopplung des Lichtes der Beleuchtungsquelle **1** wird ein Strahlteiler **6** verwendet, der gleichzeitig als Sperrfilter, zum Schutz des Beobachters vor zu hoher Bestrahlung mit dem kurzwelligen Beleuchtungslicht, dient. Das erzeugte Beleuchtungsmuster wird durch eine Projektionsoptik **7** auf den Strahlteiler **6,** der als Spiegel oder Würfel ausgeführt sein kann, gelenkt und über das im Beobachtungsstrahlengang angeordnete Objektiv **9** direkt in das Patientenauge **8** abgebildet. Dieses im Beobachtungsstrahlengang angeordnete Objektiv **9** ist vorzugsweise im UV- und/oder VIS- Bereich des Lichtes korrigiert. Um eine ungehinderte gleichzeitige Beobachtung des Patientenauges **8** durch den Beobachter zu gewährleisten, ist der Strahlteiler **6** dabei für Licht aus dem VIS-Bereich durchlässig. Die Rückseite des Strahlteilers **6** ist zum Schutz gegen zu hohe kurzwelliger Bestrahlung des Beobachters als Sperrfilter ausgebildet.

In einer weiteren Ausgestaltung der Beleuchtungs- und Bestrahlungseinheit für verschiedene ophthalmologische Geräte ist die Beleuchtungsquelle **1** als separates Bauteil, außerhalb der eigentlichen Beleuchtungseinheit angeordnet. Die Verbindung zu den Mitteln zur Erzeugung spezieller Beleuchtungsmuster und/oder Profile, die sich in der Beleuchtungseinheit befinden, wird dabei über Lichtleiter hergestellt.

Die Beleuchtungs- und Bestrahlungseinheit kann weiterhin über eine Kontrolleinheit zur Überwachung der Strahlendosis, zur Aufzeichnung der Bestrahlungsmuster und zur Registrierung der bestrahlten Positionen, verfügen. Die Kontrolleinheit weist dabei vorzugsweise ein oder mehrere Schnittstelle(n) **10** zum Datentransfer auf. Als Kontrolleinheit kann hierbei auch ein Computer eingesetzt werden, der beispielsweise in den Fuß **11** der Spaltlampe integriert sein kann.

Für andere Anwendungen, die beispielsweise die photodynamische Therapie (PDT) ist es vorteilhaft, dass von der Beleuchtungsquelle **1** schmalbandiges, langwelliges Licht, vorzugsweise um 690nm, ausgesendet wird. Entsprechend **Figur 1** werden die erzeugten Lichtbündel mit der Kondensorgruppe **2** auf die Mittel zur Erzeugung von Beleuchtungsmustern und/oder Profile gelenkt. Diese Mittel können hierbei ebenfalls feste oder austauschbare, optische Filter **4** und/oder Blenden **5,** oder aber auch opto-elektronische Lichtmodulatoren **3** sein.

In **Figur 1** ist eine besondere Ausführungsform der Beleuchtungs- und Bestrahlungseinheit für eine Spaltlampe dargestellt. Die Beleuchtungs- und Bestrahlungseinheit ist hierbei in einem separaten Gehäuse als mögliche Zusatz- oder Nachrüsteinheit für verschiedene ophthalmologische Geräte vorgesehen. Auch bei dieser Ausführungsform wird das bewährte Design bekannter ophthalmologische Geräte beibehalten.

Durch die Verwendung von Filtern **4,** Blenden **5** und insbesondere opto-elektronischen Lichtmodulatoren **3** können beliebige Muster, Profile und Verteilungen erzeugt und damit unterschiedlichste Wirkungen am oder im Patientenauge **8** hervorgerufen werden.

Eine zusätzlich vorhandene Eye-Tracker-Einheit (nicht dargestellt) dient der Überwachung möglicher Augenbewegungen, der Kontrolle der Ausrichtung der Beleuchtungsmuster auf die zu bestrahlenden Bereiche während der Bestrahlung und/oder einer Nachführung der Beleuchtungsmuster. Die Nachführung der Beleuchtungsmuster kann dabei sowohl mechanisch als auch optisch erfolgen. Überschreitet das Beleuchtungsmuster radial bzw. seitlich einen bestimmten vorher festgelegten Toleranzwert für eine ebenfalls vorher festgelegte Zeitdauer, kann die Bestrahlung unterbrochen und erst bei wieder Erreichen des Zielzustandes fortgesetzt werden. Außerdem kann die Zeitdauer der Bestrahlung ausgewertet werden um die jeweilige Strahlendosis nicht zu überschreiten. Es ist aber auch möglich das Beleuchtungsmuster der Augenbewegung nachzutühren.

Zur Erzeugung der entsprechenden Beleuchtungsmuster ist eine Kombination mit einer Wellenfrontmesseinheit und/oder einem Topographiesystem und/oder einem Augenachslängenmessgerät besonders vorteilhaft. Dabei können sich die Wellenfrontmesseinheit und/oder das Topographiesystem und/oder das Augenachslängenmessgerät mit der Beleuchtungs- und Bestrahlungseinheit in einem gemeinsamen Gehäuse befinden oder auch in den Fuß 11 der Spaltlampe integriert sein.

Die Einkopplung der erzeugten Beleuchtungsmuster und -profile in das Beobachtungssystem, z. B. das Beobachtungsmikroskop einer Spaltlampe, wirkt sich bei der vorgeschlagenen Lösung als besonders vorteilhaft aus. Dadurch ist eine ungehinderte Arbeit mit den bewährten mechanisch-optischen, kompakten Designs ophthalmologischer Geräte möglich. Außerdem hat dies den Vorteil, dass die Beleuchtungsstrahlen mit den Beobachtungsstrahlen koaxial verlaufen. Wird die Einkoppelstelle der Beleuchtungsstrahlung in den parallelen Strahlengang eines Galileisystems gelegt so treten, im Gegensatz zu den außerhalb des Beobachtungsstrahlenganges verwendeten Linksystemen, kaum Abbildungsfehler, wie beispielsweise Astigmatismus auf. Zudem ist bei der Verwendung äußerer Spiegel mit dem Auftreten von Astigmatismus und einem möglichen Bildversatz, sowie zusätzlicher Verschmutzung zu rechnen.

Die vorgeschlagene technische Lösung kann auch als modular ansetzbare Einheit, zum nachträglichen Einbau in den parallelen Strahlengang eines ophthalmologischen Gerätes konzipiert werden. Dazu wird ein bereits im jeweiligen ophthalmologische Gerät vorhandener Strahlteiler genutzt. Die Beleuchtungsund Bestrahlungseinheit kann also als eigenständige Einheit oder als Zusatzeinheit für verschiedene ophthalmologische Geräte, wie Spaltlampen, Funduskameras, Laserscanner, Ophthalmoskope und OPMI-Geräte verwendet werden.

## Patentansprüche

1. Beleuchtungs- und Bestrahlungseinheit für Spaltlampen-Mikroskope, bestehend aus eines Beleuchtungsquelle (1), Mitteln (3) zur Erzeugung spezieller Beleuchtungsmuster und/oder Profile und Mitteln (6) zur Einkopplung des Lichtes der Beleuchtungsquelle, wobei die Mittel zur Erzeugung spezieller Beleuchtungsmuster und/oder Profile opto-elektronische Lichtmodulatoren sind und **dadurch gekennzeichnet, dass** die Mittel zur Einkopplung des Lichtes der Beleuchtungsquelle so angeordnet sind, dass die Einkopplung in den parallelen Strahlengang des Beobachtungssystems der Spaltlampe erfolgt.

2. Beleuchtungs- und Bestrahlungseinheit nach Anspruch 1, **dadurch gekennzeichnet, dass** die opto-elektronischen Lichtmodulatoren vom reflektierenden, transmissiven oder selbstleuchtenden Typ sein können.

3. Beleuchtungs- und Bestrahlungseinheit nach Anspruch 1, **dadurch gekennzeichnet, dass** zusätzlich eine Kontrolleinheit, zur Überwachung der Strahlendosis, zur Aufzeichnung der Bestrahlungsmuster und zur Registrierung der bestrahlten Positionen vorhanden ist.

4. Beleuchtungs- und Bestrahlungseinheit nach Anspruch 3, **dadurch gekennzeichnet, dass** die Kontrolleinheit über ein oder mehrere Schnittstelle(n) zum Datentransfer verfügt.

5. Beleuchtungs- und Bestrahlungseinheit nach Anspruch 1, **dadurch gekennzeichnet, dass** die Beleuchtungsquelle schmalbandiges Licht im kurzwelligen Bereich, vorzugsweise um 365nm, erzeugt.

6. Beleuchtungs- und Bestrahlungseinheit nach Anspruch 1, **dadurch gekennzeichnet, dass** die Beleuchtungsquelle schmalbandiges Licht im langwelligen Bereich, vorzugsweise um 690nm, erzeugt.

7. Beleuchtungs- und Bestrahlungseinheit nach Anspruch 1, **dadurch gekennzeichnet, dass** als Mittel zur Einkopplung des Lichtes der Beleuchtungsquelle ein Strahlteiler verwendet wird, der gleichzeitig als Sperrfilter, zum Schutz des Beobachters vor zu hoher Bestrahlung mit dem Beleuchtungslicht, dienen kann.

8. Beleuchtungs- und Bestrahlungseinheit nach Anspruch 1, **dadurch gekennzeichnet, dass** die Beleuchtungsquelle nicht innerhalb der Beleuchtungseinheit, sondern als separates Bauteil angeordnet und über einen Lichtleiter mit den Mitteln zur Erzeugung spezieller Beleuchtungsmuster und/oder Profile verbunden ist.

9. Beleuchtungs- und Bestrahlungseinheit nach Anspruch 1, **dadurch gekennzeichnet, dass** zusätzlich eine Eye-Tracker-Einheit zur Kontrolle der Ausrichtung der Beleuchtungsmuster auf die zu bestrahlenden Bereiche während der Bestrahlung und/oder zur Nachführung vorhanden ist.

10. Beleuchtungs- und Bestrahlungseinheit nach Anspruch 1, **dadurch gekennzeichnet, dass** diese als modular ansetzbare Einheit, zum nachträglichen Einbau in den parallelen Strahlengang einer Spaltlampe konzipiert ist.

11. Beleuchtungs- und Bestrahlungseinheit nach Anspruch 1, **dadurch gekennzeichnet, dass** diese in Kombination mit Baugruppen, wie einer Wellenfrontmesseinheit und/oder einem Topographiesystem und/oder einem Augenachslängenmessgerät für verschiedene ophthalmologische Geräte einsetzbar ist.

12. Beleuchtungs- und Bestrahlungseinheit nach Anspruch 12, **dadurch gekennzeichnet, dass** diese mit anderen Baugruppen, wie einer Wellenfrontmesseinheit und/oder einem Topographiesystem und/oder einem Augenachslängenmessgerät in einem gemeinsamen Gehäuse angeordnet sein kann.

## Claims

1. Illumination and irradiation unit for slit-lamp microscopes, consisting of
an illumination source (1), means (3) for generating specific illumination patterns and/or profiles, and means (6) for coupling-in the light from the illumination source, wherein the means for generating specific illumination patterns and/or profiles are optoelectronic light modulators and **characterized in that** the means for coupling-in the light from the illumination source are arranged in such a way that coupling-in is effected in the parallel beam path of the observation system of the slit lamp.

2. Illumination and irradiation unit according to Claim 1, **characterized in that** the optoelectronic light modulators may be of the reflective, transmissive or self-luminous type.

3. Illumination and irradiation unit according to Claim 1, **characterized in that** there is additionally a control unit for monitoring the radiation dose, for recording the irradiation pattern and for registering the irradiated positions.

4. Illumination and irradiation unit according to Claim 3, **characterized in that** the control unit comprises one or more interfaces for data transfer.

5. Illumination and irradiation unit according to Claim 1, **characterized in that** the illumination source produces narrowband light in the short wavelength range, preferably around 365 nm.

6. Illumination and irradiation unit according to Claim 1, **characterized in that** the illumination source produces narrowband light in the long wavelength range, preferably around 690 nm.

7. Illumination and irradiation unit according to Claim 1, **characterized in that** a beam splitter is used as means for coupling-in the light from the illumination source, it being possible for said beam splitter to simultaneously serve as band-elimination filter for protecting the observer from excessive irradiation with the illumination light.

8. Illumination and irradiation unit according to Claim 1, **characterized in that** the illumination source is not arranged within the illumination unit but is instead arranged as a separate component and connected by way of an optical waveguide to the means for generating specific illumination patterns and/or profiles.

9. Illumination and irradiation unit according to Claim 1, **characterized in that**, additionally, an eye tracker unit for controlling the alignment of the illumination pattern on the regions to be irradiated during the irradiation and/or for tracking purposes is present.

10. Illumination and irradiation unit according to Claim 1, **characterized in that** it is designed as a unit which is placeable in modular fashion for a subsequent installation into the parallel beam path of a slit lamp.

11. Illumination and irradiation unit according to Claim 1, **characterized in that** it is usable in combination with assemblies such as a wavefront measuring unit and/or a topography system and/or an eye axis length measuring appliance for various ophthalmological appliances.

12. Illumination and irradiation unit according to Claim 12, **characterized in that** it may be arranged in a common housing with other assemblies such as a wavefront measuring unit and/or a topography system and/or an eye axis length measuring appliance.

## Revendications

1. Unité d'éclairage et d'irradiation pour microscope à lampes à fente, composée d'une source d'éclairage (1), de moyens (3) servant à générer un modèle et/ou des profils d'éclairage spéciaux et de moyens (6) servant à l'injection de la lumière de la source d'éclairage, les moyens servant à générer un modèle et/ou des profils d'éclairage spéciaux étant des modulateurs de lumière optoélectroniques, et **caractérisée en ce que** les moyens servant à l'injection de la lumière de la source d'éclairage sont disposés de telle sorte que l'injection s'effectue dans le trajet des faisceaux parallèles du système d'observation de la lampe à fente.

2. Unité d'éclairage et d'irradiation selon la revendication 1, **caractérisée en ce que** les modulateurs de lumière optoélectroniques peuvent être du type réfléchissant, transmissif ou autolumineux.

3. Unité d'éclairage et d'irradiation selon la revendication 1, **caractérisée en ce qu'**il existe en plus une unité de commande destinée à surveiller la dose de rayons, à enregistrer le modèle d'irradiation et à enregistrer les positions irradiées.

4. Unité d'éclairage et d'irradiation selon la revendication 3, **caractérisée en ce que** l'unité de commande dispose d'une ou de plusieurs interfaces pour le transfert de données.

5. Unité d'éclairage et d'irradiation selon la revendication 1, **caractérisée en ce que** la source d'éclairage génère de la lumière à bande étroite dans la plage des courtes longueurs d'onde, de préférence de l'ordre de 365 nm.

6. Unité d'éclairage et d'irradiation selon la revendication 1, **caractérisée en ce que** la source d'éclairage génère de la lumière à bande étroite dans la plage des grandes longueurs d'onde, de préférence de l'ordre de 690 nm.

7. Unité d'éclairage et d'irradiation selon la revendication 1, **caractérisée en ce qu'**un séparateur de faisceau est utilisé en tant que moyens servant à l'injection de la lumière de la source d'éclairage, lequel peut en même temps servir de filtre de blocage pour protéger l'observateur d'une irradiation excessive avec la lumière d'éclairage.

8. Unité d'éclairage et d'irradiation selon la revendication 1, **caractérisée en ce que** la source d'éclairage n'est pas disposée à l'intérieur de l'unité d'éclairage, mais sous la forme d'un composant séparé et est reliée par le biais d'une fibre optique aux moyens servant à générer un modèle et/ou des profils d'éclairage spéciaux.

9. Unité d'éclairage et d'irradiation selon la revendication 1, **caractérisée en ce qu'**il existe en plus une unité d'oculométrie destinée à contrôler l'orientation du modèle d'éclairage sur les zones à irradier pendant l'irradiation et/ou destinée au suivi.

10. Unité d'éclairage et d'irradiation selon la revendication 1, **caractérisée en ce que** celle-ci est conçue sous la forme d'une unité à montage modulaire destinée à être installée postérieurement dans le trajet des faisceaux parallèles d'une lampe à fente.

11. Unité d'éclairage et d'irradiation selon la revendication 1, **caractérisée en ce que** celle-ci peut être utilisée en combinaison avec des sous-ensembles comme une unité de mesure de front d'onde et/ou un système de topographie et/ou un appareil de mesure de la longueur de l'axe oculaire pour différents appareils ophtalmologiques.

12. Unité d'éclairage et d'irradiation selon la revendication 12, **caractérisée en ce que** celle-ci peut être disposée dans un boîtier commun avec d'autres sous-ensembles, comme une unité de mesure de front d'onde et/ou un système de topographie et/ou un appareil de mesure de la longueur de l'axe oculaire.
